# EUROPÄISCHE PATENTANMELDUNG

(11) **EP 4 847 121 A1**
(43) Veröffentlichungstag der Anmeldung: **02.12.2026**
(21) Anmeldenummer: 26179874.8
(22) Anmeldetag: 20.05.2026
(51) Int. Cl.: B01D 63/02, A61M 1/14, A61M 1/16, A61M 1/34, A61M 1/36, A61M 60/38, B01D 61/24, B01D 61/28, B01D 61/32, B01D 65/10, G01N 27/08

(54) **FILTER FÜR EINE EXTRAKORPORALE BLUTBEHANDLUNGSVORRICHTUNG, EXTRAKORPORALE BLUTBEHANDLUNGSVORRICHTUNG UND VERFAHREN ZUM ERKENNEN EINES FILTERS IN EINER EXTRAKORPORALEN BLUTBEHANDLUNGSVORRICHTUNG**

(30) Priorität: 28.05.2025 DE 102025120947
(71) Anmelder: B. Braun Avitum AG, 34212 Melsungen (DE)
(72) Erfinder: Janik, Waldemar, 34212 Melsungen (DE); Broeker, Bjoern, 34355 Staufenberg (DE); Lecke, Tobias, 34123 Kassel (DE); Meibaum, Joern, 34225 Baunatal (DE)
(74) Vertreter: Patentanwälte Ruff, Wilhelm, Beier, Dauster & Partner mbB

(57) **Zusammenfassung**

Die Erfindung betrifft einen Filter für eine extrakorporale Blutbehandlungsvorrichtung, wobei ein Inneres des Filters mit einer Markierungssubstanz versehen ist, die durch eine Messeinrichtung der extrakorporalen Blutbehandlungsvorrichtung nachweisbar ist.

Weiter betrifft die Erfindung eine extrakorporale Blutbehandlungsvorrichtung, die Verwendung des Filters für eine extrakorporale Blutbehandlungsvorrichtung sowie ein Verfahren zum Erkennen eines Filters in einer extrakorporalen Blutbehandlungsvorrichtung.

## Beschreibung

### ANWENDUNGSGEBIET UND STAND DER TECHNIK

Die Erfindung betrifft einen Filter für eine extrakorporale Blutbehandlungsvorrichtung, eine extrakorporale Blutbehandlungsvorrichtung sowie ein Verfahren zum Erkennen eines Filters in einer extrakorporalen Blutbehandlungsvorrichtung.

Für eine Dialyse, insbesondere Hämodialyse, weist eine extrakorporale Blutbehandlungsvorrichtung wenigstens einen Flüssigkeitsfilter auf. Dieser Filter wird von einer Dialysierflüssigkeit durchströmt und verhindert aufgrund seines Porendurchmessers und seines hohen Rückhaltevermögens für mikrobiologische Verunreinigungen die Passage mikrobieller Substanzen, wodurch eine hochreine Dialysierflüssigkeit gewährleistet wird.

Der Flüssigkeitsfilter ist auf die extrakorporale Blutbehandlungsvorrichtung abgestimmt und sorgt dafür, dass im Zusammenspiel aller Komponenten der extrakorporalen Blutbehandlungsvorrichtung eine sichere und adäquate Dialysetherapie durchgeführt werden kann. Die Verwendung von für die extrakorporale Blutbehandlungsvorrichtung zugelassenen Komponenten ist entscheidend, um die Sicherheit und Wirksamkeit der Dialysetherapie zu gewährleisten. Dies ist regulatorisch vorgeschrieben und Teil von Produktanforderungen für jede extrakorporale Blutbehandlungsvorrichtung.

Die Verwendung von Wettbewerbsprodukten in einer extrakorporalen Blutbehandlungsvorrichtung ist in der Regel möglich, jedoch häufig insoweit nicht getestet und abgestimmt, so dass eine Sicherstellung der Dialysesicherheit sowie der Wirksamkeit der Dialyse von dem ursprünglichen Hersteller der extrakorporalen Blutbehandlungsvorrichtung nicht gewährleistet werden kann.

Je nach Hersteller liegen die Betriebszeiten in einer Größenordnung von ca. drei Monaten oder erlauben lediglich eine begrenzte Anzahl von Einzelbehandlungen oder Betriebsstunden. Wird eines der vorgenannten Kriterien erreicht, muss der Filter ausgetauscht werden, weswegen man in diesem Zusammenhang auch von sogenannten Wechselkriterien spricht.

Grundsätzlich problematisch ist, dass der Schritt des Filterwechsels umgangen werden kann, indem der Wechsel beispielsweise manuell bestätigt und/oder der verbrauchte Filter weiterhin verwendet wird. Ferner besteht das Risiko, dass ein im Zusammenhang einer jeweiligen extrakorporalen Blutbehandlungsvorrichtung nicht getesteter Filter zum Einsatz kommt. Dies könnte unter Umständen dazu führen, dass eine hochreine Dialysierflüssigkeit nicht mehr garantiert werden kann, was wiederum die Patientensicherheit gefährden könnte.

Die vorgenannten Probleme sowie Risiken bestehen sinngemäß auch in Bezug auf den Dialysator als weiteren Filtertyp einer extrakorporalen Blutbehandlungsvorrichtung. Der Dialysator ist als Einmalprodukt ausgelegt und sollte nach erfolgter Dialysebehandlung fachgerecht entsorgt werden. Allerdings besteht auch im Falle des Dialysators das Risiko einer Mehrfachbenutzung.

### AUFGABE UND LÖSUNG

Der vorliegenden Erfindung liegt die Aufgabe zugrunde, einen Filter für eine extrakorporale Blutbehandlungsvorrichtung sowie eine extrakorporale Blutbehandlungsvorrichtung bereitzustellen, die die eingangs aus dem Stand der Technik bekannten Nachteile vermeiden. Weiter liegt der Erfindung die Aufgabe zugrunde, ein Verfahren zum Erkennen eines Filters in einer extrakorporalen Blutbehandlungsvorrichtung bereitzustellen.

Diese Aufgaben werden erfindungsgemäß gelöst durch einen Filter gemäß unabhängigem Anspruch 1, eine extrakorporale Blutbehandlungsvorrichtung gemäß Anspruch 12, die Verwendung eines Filters gemäß Anspruch 13 sowie ein Verfahren zum Erkennen eines Filters in einer extrakorporalen Blutbehandlungsvorrichtung gemäß Anspruch 14. Bevorzugte Ausgestaltungen der Erfindung sind Gegenstand der abhängigen Ansprüche. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung einen Filter für eine extrakorporale Blutbehandlungsvorrichtung. Bei der extrakorporalen Blutbehandlungsvorrichtung handelt es sich vorzugsweise um eine Dialysevorrichtung, insbesondere Hämodialyse- oder Peritonealdialysevorrichtung.

Der Filter zeichnet sich besonders dadurch aus, dass ein Inneres des Filters mit einer Markierungssubstanz versehen ist, die durch eine Messeinrichtung der extrakorporalen Blutbehandlungsvorrichtung nachweisbar ist.

Die Erfindung beruht insbesondere auf dem überraschenden Befund, dass die Einwechslung von nicht getesteten und/oder nicht regulatorisch vorgeschriebenen Filtern in eine extrakorporale Blutbehandlungsvorrichtung durch eine Markierung eines Inneren eines für eine extrakorporale Blutbehandlungsvorrichtung tauglichen Filters mit einer Substanz vermieden werden kann. Dies leistet einen entscheidenden Beitrag zur Aufrechterhaltung und/oder Erhöhung der Qualität einer extrakorporalen Blutbehandlung sowie insbesondere der Patientensicherheit. Weiter ist von Vorteil, dass eine solche Markierung, wie im Folgenden noch eingehender erläutert werden wird, nicht nur eine Erkennung des Filters als solchen, sondern auch eine Identifizierung von dessen Merkmalen, wie beispielsweise Form, Größe, Füllvolumen, Porendurchmesser einer Membran des Filters, Material einer Membran des Filters und/oder Oberfläche einer Membran des Filters, ermöglicht.

Bei der Messeinrichtung der extrakorporalen Blutbehandlungsvorrichtung kann es sich insbesondere um eine optische Messeinrichtung, vorzugsweise Extinktions- oder Absorptionsmesseinrichtung, handeln.

Der Ausdruck "Extinktionsmesseinrichtung" bedeutet im Sinne der vorliegenden Erfindung eine Messeinrichtung, die zum Messen einer Extinktion eingerichtet ist.

Unter dem Ausdruck "Extinktion" soll im Sinne der vorliegenden Erfindung in Übereinstimmung mit dem fachmännischen Verständnis die optische Dichte verstanden werden, die ein Maß für die Abschwächung von Licht nach dessen Durchqueren eines Mediums darstellt. Die Extinktion lässt sich aus dem logarithmischen Verhältnis von in das Medium einfallender Strahlung zu der aus dem Medium austretenden Strahlung berechnen.

Unter dem Ausdruck "Transmission" soll - ebenfalls in Übereinstimmung mit dem fachmännischen Verständnis - im Sinne der vorliegenden Erfindung eine Größe für die Durchlässigkeit eines Mediums für Licht verstanden werden. Die Transmission ergibt sich aus dem Verhältnis einer nach Durchqueren eines Mediums aus dem Medium austretenden Strahlung zu einer in das Medium einfallenden Strahlung.

Alternativ kann es sich bei der Messeinrichtung der extrakorporalen Blutbehandlungsvorrichtung um eine Leitfähigkeitsmesseinrichtung, d. h. um eine Messeinrichtung, die zum Messen einer Leitfähigkeit eingerichtet ist, handeln. Bevorzugt ist die Leitfähigkeitsmesseinrichtung zum Messen einer Leitfähigkeit eines wässrigen Fluids, insbesondere einer salz- oder elektrolythaltigen Lösung, vorzugsweise eines basischen Fluids, eines sauren Fluids und/oder einer Dialysierflüssigkeit, eingerichtet.

Unter dem Ausdruck "basisches Fluid" soll im Sinne der vorliegenden Erfindung ein wässriges Fluid verstanden werden, das Bicarbonationen, insbesondere in Form von Natriumhydrogencarbonat (NaHCO₃), enthält. Üblicherweise handelt es sich bei dem basischen Fluid um eine wässrige Natriumhydrogencarbonat-Lösung. Das basische Fluid kann auch als basisches Konzentrat bezeichnet werden.

Unter dem Ausdruck "saures Fluid" soll im Sinne der vorliegenden Erfindung ein wässriges Fluid verstanden werden, das Natrium-, Kalium-, Calcium- und Magnesiumionen sowie eine Säure, vorzugsweise Essigsäure, enthält. Zusätzlich kann Glucose in dem sauren Fluid enthalten sein. Üblicherweise handelt es sich bei dem sauren Fluid um eine wässrige Lösung, die Natriumchlorid, Kaliumchlorid, Magnesiumchlorid, Calciumchlorid, Glucose und Essigsäure (oder eine andere Säure wie Zitronensäure) enthält.

Unter dem Ausdruck "Dialysierflüssigkeit" soll im Sinne der vorliegenden Erfindung eine Flüssigkeit für die Dialyse, insbesondere Hämodialyse und/oder Peritonealdialyse, verstanden werden, die aus Wasser, d. h. hochreinem Wasser (sogenanntes Permeat oder Osmosewasser), einem basischen Fluid und einem sauren Fluid hergestellt oder herstellbar ist.

In Ausgestaltung der Erfindung ist ein Strömungsbereich des Filters, der, insbesondere beim bestimmungsgemäßen Gebrauch des Filters oder bei Inbetriebnahme der extrakorporalen Blutbehandlungsvorrichtung, von einem Fluid durchströmbar ist, mit der Markierungssubstanz versehen. Bei dem Fluid handelt es sich vorzugsweise um ein wässriges Fluid, insbesondere um Wasser (bevorzugt Osmosewasser oder Permeat), ein basisches Fluid, ein saures Fluid oder eine Dialysierflüssigkeit. Bei dem Strömungsbereich kann es sich beispielsweise um die Blut- und/oder Dialysatseite, d.h. die Blut- und/oder Dialysatkammer, eines als Dialysator gestalteten Filters handeln.

In weiterer Ausgestaltung der Erfindung ist eine Innenoberfläche, insbesondere eine Oberfläche einer Membran, des Filters mit der Markierungssubstanz versehen, insbesondere beschichtet. Die Membran ist vorzugsweise als Hohlfasermembran, d. h. als Membran, die wenigstens eine Hohlfasermembran aufweist oder aus wenigstens einer Hohlfasermembran besteht, gestaltet sein.

Der Ausdruck "Hohlfasermembran" bedeutet im Sinne der vorliegenden Erfindung eine hohlfaserförmig gestaltete Membran.

Der Ausdruck "wenigstens eine Hohlfasermembran" kann im Sinne der vorliegenden Erfindung eine, d. h. nur eine, Hohlfasermembran oder eine Vielzahl von, d. h. zwei oder mehr, Hohlfasermembranen bedeuten.

Bei der bzw. den in den vorherigen Absätzen beschriebenen Membran bzw. Membranen handelt es sich vorzugsweise um eine semipermeable Membran bzw. um semipermeable Membranen.

In weiterer Ausgestaltung der Erfindung umfasst die Markierungssubstanz eine mit Hilfe eines wässrigen Fluids entfernbare Substanz, vorzugsweise eine wenigstens teilweise, insbesondere nur teilweise oder vollständig, wasserlösliche Substanz. Insbesondere kann es sich bei der Markierungssubstanz um eine mit Hilfe eines wässrigen Fluids entfernbare Substanz, vorzugsweise um eine wenigstens teilweise, insbesondere nur teilweise oder vollständig, wasserlösliche Substanz, handeln. Dies hat den Vorteil, dass die Markierung des Filters nur von temporärer, d.h. vorübergehender, Natur ist und insbesondere das Risiko vermieden werden kann, dass ein Patient bei der extrakorporalen Blutbehandlung mit der Markierungssubstanz in Kontakt gerät. So ermöglicht diese Ausgestaltung der Erfindung ein Erkennen des Filters noch vor Durchführung einer extrakorporalen Blutbehandlung, beispielsweise indem der Filter in einem Bypass-Betrieb der extrakorporalen Blutbehandlungsvorrichtung mit dem wässrigen Fluid gespült wird. Bei dem wässrigen Fluid kann es sich insbesondere um Wasser (bevorzugt Permeat oder Osmosewasser), basisches Fluid, saures Fluid oder eine Dialysierflüssigkeit handeln.

Bevorzugt liegt die Markierungssubstanz in fester, insbesondere kristalliner und/oder pulverförmiger, Form vor.

Alternativ kann die Markierungssubstanz in wässrig gelöster Form, d. h. als Bestandteil einer wässrigen Lösung, vorliegen. In diesem Fall kann der Filter zweckmäßigerweise mit entsprechenden Verschlüssen verschlossen sein.

In weiterer Ausgestaltung der Erfindung umfasst die Markierungssubstanz ein Salz, insbesondere ein in einer Dialysierflüssigkeit und/oder im Blut, insbesondere menschlichen Blut, vorkommendes Salz. Insbesondere kann es sich bei der Markierungssubstanz um ein Salz, insbesondere ein in einer Dialysierflüssigkeit und/oder im Blut, insbesondere menschlichen Blut, vorkommendes Salz handeln.

In weiterer Ausgestaltung der Erfindung ist das Salz ausgewählt aus der Gruppe bestehend aus Natriumsalz, Kaliumsalz, Magnesiumsalz, Calciumsalz und Mischungen von wenigstens zwei der vorgenannten Salze. Vorzugsweise ist das Salz ausgewählt aus der Gruppe bestehend aus Natriumchlorid, Kaliumchlorid, Magnesiumchlorid, Calciumchlorid, Natriumhydrogencarbonat und Mischungen von wenigstens zwei der vorgenannten Salze.

In weiterer Ausgestaltung der Erfindung umfasst die Markierungssubstanz einen Farbstoff. Insbesondere kann es sich bei der Markierungssubstanz um einen Farbstoff handeln. Der Farbstoff kann insbesondere ein natürlicher, d. h. in der Natur vorkommender, Farbstoff sein. Bevorzugt absorbiert der Farbstoff Licht in einem Wellenlängenbereich von 100 nm bis 830 nm, insbesondere 527 nm bis 570 nm oder 628 nm bis 645 nm, oder Licht mit einer Wellenlänge von 465 nm.

In weiterer Ausgestaltung der Erfindung ist der Farbstoff ausgewählt aus der Gruppe bestehend aus Chlorophyll, Harnsäure, Vitamin, Thiamin (Vitamin B1), Riboflavin (Vitamin B2), Pyridoxin (Vitamin B6), Cobalamin (Vitamin B12), Ascorbinsäure (Vitamin C), Pantothensäure (Vitamin B5), Folsäure (Vitamin B9), Nicotinsäure (Vitamin B3), Biotin (Vitamin B7) und Mischungen von wenigstens zwei der vorgenannten Farbstoffe.

Weiter kann die Markierungssubstanz eine Kombination, insbesondere Mischung, von zwei oder mehr unterschiedlichen Markierungssubstanzen, insbesondere zwei oder mehr unterschiedlichen Salzen, zwei oder mehr unterschiedlichen Farbstoffen oder wenigstens einem Salz und wenigstens einem Farbstoff, umfassen. Insbesondere kann es sich bei der Markierungssubstanz um eine Kombination, insbesondere Mischung, von zwei oder mehr unterschiedlichen Markierungssubstanzen, insbesondere zwei oder mehr unterschiedlichen Salzen, zwei oder mehr unterschiedlichen Farbstoffen oder wenigstens einem Salz und wenigstens einem Farbstoff, handeln. Bezüglich in Frage kommender Markierungssubstanzen, insbesondere Salze und Farbstoffe, wird vollumfänglich auf die vorangegangenen Ausführungen Bezug genommen.

In weiterer Ausgestaltung der Erfindung ist die Markierungssubstanz von einem wenigstens teilweise, insbesondere nur teilweise oder vollständig, wasserlöslichen oder in Wasser quellbaren Material umhüllt. Durch diese Ausgestaltung ist vorteilhafterweise die Bereitstellung der wenigstens einen Markierungssubstanz in flüssiger Form erzielbar, ohne dass ein Verschluss des Filters erforderlich ist. Das wenigstens teilweise wasserlösliche Material ist vorzugsweise als Folie gestaltet. Bevorzugt handelt es sich bei dem wenigstens teilweise wasserlöslichen Material um ein Polymer, insbesondere ausgewählt aus der Gruppe bestehend aus Polyvinylalkohol, Polyethylenglykol, Gelatine, Stärke, Amylose, Amylopektin, Dextran, Methylcellulose, Hydroxymethylcellulose, Hydroxyethylcellulose, Hydroxypropylcellulose, Hydroxybutylcellulose, Hydroxypropylmethylcellulose, Carboxymethylcellulose, Hyaluronsäure, Chondroitin-4-sulfat, Chondroitin-6-sulfat, Keratansulfat, Alginat, Alginsäure, Heparin, Heparansulfat, Chitin, Chitosan, Salze der vorgenannten wenigstens teilweise wasserlöslichen Materialien, Derivate der vorgenannten wenigstens teilweise wasserlöslichen Materialien und Kombinationen, insbesondere Mischungen, von wenigstens zwei der vorgenannten wenigstens teilweise wasserlöslichen Materialien.

Vorzugsweise handelt es sich bei dem wenigstens teilweise wasserlöslichen Material um Polyvinylalkohol. Der Polyvinylalkohol kann ein Molekulargewicht von 5.000 g/mol bis 200.000 g/mol, insbesondere 10.000 g/mol bis 150.000 g/mol, bevorzugt 10.000 g/mol bis 100.000 g/mol, aufweisen.

In weiterer Ausgestaltung der Erfindung handelt es sich bei dem Filter um einen Dialysierflüssigkeitsfilter.

Unter dem Ausdruck "Dialysierflüssigkeitsfilter" soll im Sinne der vorliegenden Erfindung ein Filter verstanden werden, der zum Filtern von in Fluidleitungen einer extrakorporalen Blutbehandlungsvorrichtung strömenden wässrigen Fluiden eingerichtet ist.

In weiterer Ausgestaltung der Erfindung handelt es sich bei dem Filter um einen Dialysator. Der Dialysator kann als Kapillardialysator oder Plattendialysator gestaltet sein. Bevorzugt ist der Dialysator jedoch als Kapillardialysator gestaltet, d. h. als Dialysator, in dem eine große Anzahl von Hohlfasern, insbesondere bis zu 18.000 Hohlfasern, vorzugsweise parallel, angeordnet sind. Die Wand der Hohlfasern wird durch eine semipermeable Membran gebildet, die für Wasser und darin gelöste Substanzen bis zu einer mittleren Molekülgröße durchlässig ist, für die Blutzellen und größere Proteine, wie beispielsweise Albumin, dagegen nicht. Über die semipermeablen Membranen erfolgt der Stofftransport der gelösten Substanzen in Blut und Dialysat, entweder durch selektive Diffusion oder Konvektion. Die Ultrafiltration, also der Transport von Wasser vom Blut in den Dialysatkreislauf, wird durch Anlegen eines Druckunterschieds über die Membran erzeugt. Diese Druckdifferenz, der sogenannte Transmembrandruck, setzt sich aus der statischen Druckdifferenz zwischen Blut und Dialysat sowie einem osmotischen Druck, der im Wesentlichen durch die Konzentrationsunterschiede von Albumin auf beiden Seiten der Membran entsteht, zusammen.

Gemäß einem zweiten Aspekt betrifft die Erfindung eine extrakorporale Blutbehandlungsvorrichtung, welche einen Filter gemäß erstem Erfindungsaspekt aufweist.

Bezüglich weiterer Merkmale und Vorteile der extrakorporalen Blutbehandlungsvorrichtung sowie des Filters wird zur Vermeidung von Wiederholungen vollständig auf die im Rahmen des ersten

Erfindungsaspekts gemachten Ausführungen Bezug genommen. Die dort in Bezug auf die extrakorporale Blutbehandlungsvorrichtung sowie den Filter beschriebenen Merkmale und Vorteile gelten sinngemäß auch für eine extrakorporale Blutbehandlungsvorrichtung gemäß zweitem Erfindungsaspekt.

Gemäß einem dritten Aspekt betrifft die Erfindung die Verwendung eines Filters gemäß erstem Erfindungsaspekt für eine extrakorporale Blutbehandlungsvorrichtung.

Bezüglich weiterer Merkmale und Vorteile des Filters sowie der extrakorporalen Blutbehandlungsvorrichtung wird zur Vermeidung von Wiederholungen ebenfalls vollständig auf die im Rahmen des ersten Erfindungsaspekts gemachten Ausführungen Bezug genommen. Die dort in Bezug auf den Filter sowie die extrakorporale Blutbehandlungsvorrichtung beschriebenen Merkmale und Vorteile gelten sinngemäß auch für die Verwendung eines Filters gemäß drittem Erfindungsaspekt.

Gemäß einem vierten Aspekt betrifft die Erfindung ein Verfahren zum Erkennen eines Filters gemäß erstem Erfindungsaspekt in einer extrakorporalen Blutbehandlungsvorrichtung. Das Verfahren weist, insbesondere in zeitlicher Reichenfolge, die nachfolgenden Schritte auf:

| | |
|---|---|
| S1) | Einbauen eines Filters in die extrakorporale Blutbehandlungsvorrichtung, |
| S2) | erstmaliges Durchspülen des eingebauten Filters mit einem wässrigen Fluid, |
| S3) | Aufnehmen eines Messsignals mit Hilfe einer Messeinrichtung der extrakorporalen Blutbehandlungsvorrichtung, wobei das Messsignal eine chemische und/oder physikalische Eigenschaft der Markierungssubstanz repräsentiert, |
| S4) | Auswerten des aufgenommenen Messsignals und |
| S5) | Erkennen anhand des ausgewerteten Messignals, ob es sich bei dem eingebauten Filter um einen Filter gemäß erstem Erfindungsaspekt handelt. |

Der nach Durchführen von Schritt S1) eingebaute Filter ersetzt vorzugsweise einen anderen Filter der extrakorporalen Blutbehandlungsvorrichtung. Alternativ kann durch den Schritt S1) erstmalig ein Filter in die extrakorporale Blutbehandlungsvorrichtung eingebaut werden.

Der im Zusammenhang des Schritts S2) verwendete Ausdruck "erstmaliges Durchspülen" bedeutet im Sinne der vorliegenden Erfindung, dass der eingebaute Filter zum ersten Mal mit einem wässrigen Fluid durchgespült wird.

Zum Durchführen von Schritt S2) kann als Fluid insbesondere Wasser (bevorzugt Osmosewasser oder Permeat), basisches Fluid, saures Fluid oder Dialysierflüssigkeit verwendet werden.

Bei der Messeinrichtung gemäß Schritt S3) handelt es sich vorzugsweise um eine optische Messeinrichtung, insbesondere um eine Extinktions- oder Absorptions-Messeinrichtung, oder eine Leitfähigkeitsmesseinrichtung. Dementsprechend bevorzugt handelt es sich bei der chemischen und/oder physikalischen Eigenschaft der Markierungssubstanz um eine optische Eigenschaft, insbesondere die Extinktion oder Absorption, oder die Leitfähigkeit der Markierungssubstanz.

Das Verfahren kann ferner vor dem Schritt S1) einen weiteren Schritt S0) aufweisen:
- Ausgeben eines Aufforderungshinweises zum Filterwechsel, wenn wenigstens ein Kriterium für den Filterwechsel, d. h. wenigstens ein Wechselkriterium, erreicht ist.

Das wenigstens eine Kriterium für einen Filterwechsel bzw. das wenigstens eine Wechselkriterium kann beispielsweise eine definierte Anzahl von mit Hilfe der extrakorporalen Blutbehandlungsvorrichtung durchzuführenden Einzelbehandlungen und/oder eine definierte Anzahl von Betriebsstunden sein.

Bevorzugt weist das Verfahren nach dem Schritt S5) ferner einen Schritt S6) auf:
- Identifizieren eines Merkmals des eingebauten Filters anhand des ausgewerteten Messsignals, wenn der Schritt S5) ergibt, dass es sich bei dem eingebauten Filter um einen erfindungsgemäßen Filter handelt.

Das wenigstens eine Merkmal des Filters kann beispielswiese ausgewählt sein aus der Gruppe bestehend aus Form, Größe, Füllvolumen, Porendurchmesser einer Membran des Filters, Material einer Membran des Filters, Oberfläche einer Membran des Filters und Kombinationen von wenigstens zwei der vorgenannten Merkmale.

Ferner kann das Verfahren nach dem Schritt S5, insbesondere nach dem Schritt S6), einen weiteren Schritt S7) aufweisen:
- Zurücksetzen wenigstens eines / des wenigstens einen Kriteriums für einen Filterwechsel.

Ferner kann das Verfahren nach dem Schritt S5) einen weiteren Schritt S8) aufweisen:
- Ausgeben eines Hinweises, dass der eingebaute Filter unbekannt ist und/oder gewechselt werden soll/muss, wenn der Schritt S5) ergibt, dass es sich bei dem eingebauten Filter nicht um einen erfindungsgemäßen Filter handelt.

Ferner kann das Verfahren nach dem Schritt S8) einen weiteren Schritt S9) aufweisen:
- Sperren der extrakorporalen Blutbehandlungsvorrichtung.

Bezüglich weiterer Merkmale und Vorteile des Filters sowie der extrakorporalen Blutbehandlungsvorrichtung wird zur Vermeidung von Wiederholungen ebenfalls vollständig auf die im Rahmen des ersten Erfindungsaspekts gemachten Ausführungen Bezug genommen. Die dort in Bezug auf den Filter sowie die extrakorporale Blutbehandlungsvorrichtung beschriebenen Merkmale und Vorteile gelten sinngemäß auch für das Verfahren gemäß viertem Erfindungsaspekt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind. Es versteht sich, dass die Erfindung hierauf nicht beschränkt sein soll.

### KURZBESCHREIBUNGEN DER FIGUREN

In den Figuren ist Folgendes schematisch gezeigt:
- Fig. 1: eine Ausführungsform eines erfindungsgemäßen Filters,
- Fig. 2: eine Ausführungsform einer erfindungsgemäßen extrakorporalen Blutbehandlungsvorrichtung,
- Fig. 3: eine weitere Ausführungsform einer erfindungsgemäßen extrakorporalen Blutbehandlungsvorrichtung,
- Fig. 4a: einen exemplarischen Messsignalverlauf im Falle eines nicht erfindungsgemäßen Filters
- Fig. 4b: einen exemplarischen Messsignalverlauf im Falle eines erfindungsgemäßen Filters und
- Fig. 5: ein Flussdiagramm einer Ausführungsform eines erfindungsgemäßen Verfahrens.

### AUSFÜHRLICHE FIGURENBESCHREIBUNG

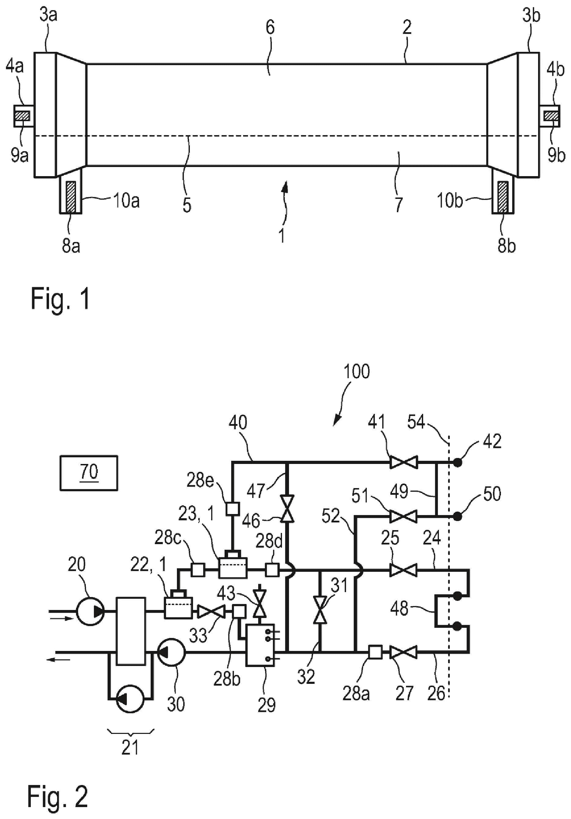

Fig. 1 zeigt schematisch eine Ausführungsform eines erfindungsgemäßen Filters 1 für eine extrakorporale Blutbehandlungsvorrichtung.

Der Filter 1 weist ein Gehäuse 2 auf. Das Gehäuse 2 ist vorzugsweise zylinderförmig gestaltet. Weiter weist der Filter 1 an gegenüberliegenden Enden des Gehäuses 2 jeweils Verschlusselemente 3a, 3b auf. Die Verschlusselemente 3a, 3b können beispielsweise kappenförmig gestaltet und mit den gegenüberliegenden Enden des Gehäuses 2 verbunden, insbesondere form- und/oder kraftschlüssig verbunden, beispielsweise verschraubt, sein. Dabei können die Enden des Gehäuses 2 konisch, insbesondere in Richtung der Verschlusselemente 3a, 3b konisch erweitert, gestaltet sein.

Die Verschlusselemente 3a, 3b weisen jeweils eine Anschlusseinrichtung 4a, 4b zum Anschließen von fluidführenden Schläuchen, insbesondere dialysierflüssigkeits-, wasser- und/oder blutführenden Schläuchen, auf.

Im Inneren des Filters 1 ist eine Vielzahl von Hohlfasermembranen 5 angeordnet. Die Hohlfasermembranen stellen jeweils eine semipermeable Membran dar, d. h. die Wandung der Hohlfasern ist von einer semipermeablen Membran gebildet. Geeignete Membranmaterialien können beispielsweise aus der Gruppe bestehend aus Polysulfon, Polyethersulfon, Celluloseacetat und Mischungen von wenigstens zwei der vorgenannten Membranmaterialien ausgewählt sein. Die Vielzahl der Hohlfasermembranen teilen das Innere des Filters 1 in eine erste Kammer 6 und eine zweite Kammer 7. Im Falle eines als Dialysator ausgebildeten Filters 1 handelt es sich bei der ersten Kammer 6 um die sogenannte Blutkammer bzw. Blutseite des Dialysators und im Falle der zweiten Kammer 7 um die Dialysatkammer bzw. Dialysatseite des Dialysators. Zur Veranschaulichung sowie der besseren Übersichtlichkeit wegen ist in Fig. 1 lediglich eine Hohlfasermembran 5 dargestellt.

Neben den Anschlusseinrichtungen 4a, 4b weist der Filter 1 zwei weitere Anschlusseinrichtungen 10a, 10b auf. Sowohl die Anschlusseinrichtungen 4a, 4b als auch die Anschlusseinrichtungen 10a, 10b können jeweils stutzenförmig gestaltet sein. Die Anschlusseinrichtungen 10a, 10b sind vorzugsweise zum Anschließen an wasserhaltige Fluide, insbesondere Dialysierflüssigkeit, führende Schläuche eingerichtet.

Der Filter 1 zeichnet sich dadurch aus, dass ein Inneres des Filters 1 mit einer Markierungssubstanz 8a, 8b, 9a, 9b versehen ist. Die Markierungssubstanz 8a, 8b, 9a, 9b ist durch eine Messeinrichtung der extrakorporalen Blutbehandlungsvorrichtung nachweisbar. Bei der Messeinrichtung kann es sich beispielsweise um eine optische Messeinrichtung, insbesondere eine Extinktions- oder Absorptionsmesseinrichtung, oder eine Leitfähigkeitsmesseinrichtung der extrakorporalen Blutbehandlungsvorrichtung handeln.

Bevorzugt ist wenigstens ein Strömungsbereich des Filters 1, der beim bestimmungsgemäßen Gebrauch bzw. bei Inbetriebnahme der extrakorporalen Blutbehandlungsvorrichtung von einem Fluid durchströmbar ist, mit der Markierungssubstanz 8a, 8b, 9a, 9b versehen.

Dementsprechend kann beispielweise - wie in Fig. 1 dargestellt - ein Inneres der Anschlusseinrichtungen 4a, 4b und ein Inneres der zwei weiteren Anschlusseinrichtungen 10a, 10b des Filters 1 oder auch nur ein Inneres wenigstens einer der beiden Anschlusseinrichtungen 4a, 4b oder auch nur ein Inneres wenigstens einer der beiden weiteren Anschlusseinrichtungen 10a, 10b mit der Markierungssubstanz 8a, 8b, 9a, 9b versehen sein. Alternativ oder in Kombination kann ein Inneres des Gehäuses 2 und/oder ein Inneres wenigstens eines der beiden Enden des Gehäuses 2 mit der Markierungssubstanz versehen sein.

Bevorzugt ist dabei eine Innenoberfläche des Filters 1, insbesondere eine Innenoberfläche des Gehäuses 2 und/oder eine Innenoberfläche wenigstens einer der beiden Anschlusseinrichtungen 4a, 4b und/oder eine Innenoberfläche wenigstens einer der beiden weiteren Anschlusseinrichtungen 10a, 10b und/oder eine Innenoberfläche wenigstens eines der beiden Enden des Gehäuses 2 und/oder eine Oberfläche der Hohlfasermembranen 5 mit der Markierungssubstanz beschichtet. Hierzu kann die Markierungssubstanz zunächst in flüssiger Form in den Filter 1 eingebracht und durch anschließendes Trocknen die Beschichtung erzeugt werden.

Bei der Markierungssubstanz 8a, 8b, 9a, 9b handelt es sich vorzugsweise um ein Salz, insbesondere um ein in einer Dialysierflüssigkeit und/oder im Blut vorkommendes Salz. Besonders bevorzugt ist das Salz ausgewählt aus der Gruppe bestehend aus Natriumchlorid, Kaliumchlorid, Magnesiumchlorid, Calciumchlorid, Natriumhydrogencarbonat und Mischungen von wenigstens zwei der vorgenannten Salze.

Alternativ oder in Kombination kann es sich bei der Markierungssubstanz 8a, 8b, 9a, 9b bevorzugt um einen Farbstoff handeln. Der Farbstoff ist vorzugsweise ausgewählt aus der Gruppe bestehend aus Chlorophyll, Harnsäure, Vitamin, Thiamin (Vitamin B1), Riboflavin (Vitamin B2), Pyridoxin (Vitamin B6), Cobalamin (Vitamin B12), Ascorbinsäure (Vitamin C), Pantothensäure (Vitamin B5), Folsäure (Vitamin B9), Nicotinsäure (Vitamin B3), Biotin (Vitamin B7) und Mischungen von wenigstens zwei der vorgenannten Farbstoffe.

Weiter kann es erfindungsgemäß bevorzugt sein, dass die Markierungssubstanz 8a, 8b, 9a, 9b von einer wenigstens teilweise wasserlöslichen, insbesondere nur teilweise wasserlöslichen oder vollständig wasserlöslichen, oder in Wasser quellbaren Umhüllung umgeben ist. Die Umhüllung ist vorzugsweise als Folie gestaltet. Bevorzugt weist die Umhüllung ein polymeres Material auf oder besteht aus einem polymeren Material. Das polymere Material kann insbesondere ausgewählt sein aus der Gruppe bestehend aus Polyvinylalkohol, Polyethylenglycol, Gelatine, Stärke, Amylose, Amylopektin, Dextran, Methylcellulose, Hydroxymethylcellulose, Hydroxyethylcellulose, Hydroxypropylcellulose, Hydroxybutylcellulose, Hydroxypropylmethylcellulose, Carboxymethylcellulose, Hyaluronsäure, Chondroitin-4-sulfat, Chondroitin-6-sulfat, Keratansulfat, Alginat, Alginsäure, Heparin, Heparansulfat, Chitin, Chitosan, Salze der vorgenannten polymeren Materialien, Derivate der vorgenannten polymeren Materialien und Kombinationen, insbesondere Mischungen, von wenigstens zwei der vorgenannten polymeren Materialien.

Besonders bevorzugt weist die Umhüllung Polyvinylalkohol auf. Der Polyvinylalkohol kann ein Molekulargewicht von 5.000 g/mol bis 200.000 g/mol, insbesondere 10.000 g/mol bis 150.000 g/mol, bevorzugt 10.000 g/mol bis 100.000 g/mol, aufweisen.

Eine Umhüllung der Markierungssubstanz 8a, 8b, 9a, 9b hat den Vorteil, dass sie ein Einbringen der Markierungssubstanz 8a, 8b, 9a, 9b in flüssiger Form ermöglicht, ohne dass ein Verschluss des Filters 1 erforderlich ist.

Alternativ kann der Filter 1 ferner abnehmbare Verschlüsse, insbesondere in Form von Kappen, aufweisen (nicht dargestellt). Insbesondere können die Anschlusseinrichtungen 4a, 4b, 10a, 10b jeweils mit einem solchen Verschluss versehen sein. Dadurch kann zum einen eine Verunreinigung des Inneren des Filters 1 sowie ein unerwünschtes Austreten der Markierungssubstanz 8a, 8b, 9a, 9b aus dem Filter 1 verhindert werden.

Fig. 2 zeigt schematisch eine Ausführungsform einer erfindungsgemäßen extrakorporalen Blutbehandlungsvorrichtung 100.

Die extrakorporale Blutbehandlungsvorrichtung 100 weist zwei erfindungsgemäße Filter, nämlich einen ersten Filter 22 und einen zweiten Filter 23, auf. Dementsprechend ist sowohl ein Inneres des ersten Filters 22 als auch ein Inneres des zweiten Filters 23 mit einer Markierungssubstanz versehen, die durch eine Messeinrichtung der extrakorporalen Blutbehandlungsvorrichtung 100 nachweisbar ist. Vorzugsweise sind der erste Filter 22 und der zweite Filter 23 gleich ausgebildet.

Bei der Markierungssubstanz kann es sich bevorzugt um ein Salz und/oder einen Farbstoff handeln. Insoweit wird auf die entsprechenden in der allgemeinen Beschreibung sowie in der Beschreibung zu Fig. 1 gemachten Ausführungen Bezug genommen.

Nach dem Einbauen der Filter 22, 23 wird die extrakorporale Blutbehandlungsvorrichtung 100 mit Wasser gespült, um Luft und mögliche Produktionsrückstände zu entfernen, die sonst einen Einfluss auf die Bilanzierung durch eine Bilanziereinheit 21 der extrakorporalen Blutbehandlungsvorrichtung 100 während einer Therapie haben könnten.

Durch das Spülen der extrakorporalen Blutbehandlungsvorrichtung 100 wird die Markierungssubstanz aus den Filtern 22, 23 entfernt, vorzugsweise gelöst und durch die Fluidleitungen der extrakorporalen Blutbehandlungsvorrichtung 100 transportiert.

Da das zum Spülen verwendete Wasser die Markierungssubstanz per se nicht enthält, steigt die Konzentration der Markierungssubstanz an, sobald sie im Wasser gelöst wird. Der Konzentrationsanstieg der Markierungssubstanz kann an unterschiedlichen Stellen im fluidleitenden System (sogenannten Hydrauliksystem) der extrakorporalen Blutbehandlungsvorrichtung 100 in Form eines Signalausschlags an einer Messvorrichtung 28 der extrakorporalen Blutbehandlungsvorrichtung 100 detektiert werden.

So ist ein Signalausschlag an der Messvorrichtung 28b und/oder an der Messvorrichtung 28c der extrakorporalen Blutbehandlungsvorrichtung 100 eindeutig dem Filter 22 zuordenbar, da die Messvorrichtungen 28b und 28c jeweils stromabwärts des Filters 22 angeordnet sind. Dagegen kann ein Signalausschlag der stromabwärts des Filters 23 angeordneten Messvorrichtungen 28d, 28e jeweils dem Filter 23 zugeordnet werden.

Insgesamt können daher beide Filter 22, 23 durch die Messvorrichtungen 28b, 28c, 28d, 28e erkannt/identifiziert werden.

Bevorzugt wird die Messvorrichtung 28a, die an einer dialysatführenden Leitung 26 der extrakorporalen Blutbehandlungsvorrichtung 100 angeordnet ist, für die Filtererkennung/- identifizierung herangezogen.

Bei den vorgenannten Messvorrichtungen 28a, 28b, 28c, 28d, 28e kann es sich um optische Messvorrichtungen, wie beispielsweise Extinktions- oder Absorptionsmessvorrichtungen, und/oder Leitfähigkeitsmessvorrichtungen handeln.

Alternativ oder in Kombination kann der Nachweis der Markierungssubstanzen der Filter 22, 23 auch über eine Messvorrichtung, vorzugsweise Leitfähigkeitsmessvorrichtung, in einem Luftabscheider 29 der extrakorporalen Blutbehandlungsvorrichtung 100 erfolgen. Durch eine geeignete Ventilstellung kann der Luftabscheider 29 mit einem wässrigen Fluid gefüllt werden, welches zuvor entweder nur durch den Filter 22 oder durch beide Filter 22, 23 geströmt ist.

Das von den vorgenannten Messvorrichtungen 28a, 28b, 28c, 28d, 28e aufgenommene Messignal kann beispielsweise von einer Steuer- und Recheneinheit 70, die entweder Bestandteil der extrakorporalen Blutbehandlungsvorrichtung 100 oder dieser zugeordnet ist, ausgewertet werden. Hierzu kann auch eine vorgeschaltete Filterung und/oder Verstärkung des aufgenommenen Messsignals vorgesehen sein.

Weiter kann die extrakorporale Blutbehandlungsvorrichtung - wie in Fig. 2 dargestellt -Pumpen 20, 30, eine dialysierflüssigkeitsführende Leitung 24, Dialysatoreingangsventile 25, 27, ein Bypassventil 31, eine Bypassleitung 32, ein Spülventil 33, eine Substituatleitung 40, ein Substituatventil 41, ein Substituatanschluss 42, ein Entlüftungsventil 43, ein Substituatbypassventil 46, eine Substituatbypassleitung 47, eine Spülbrücke 48, eine Spülleitung 49, ein Reinfusionsanschluss 50, ein Reinfusionsventil 51, eine Reinfusionsleitung 52, ein Maschinengehäuse 54 und eine Steuer- und Recheneinheit 70 aufweisen.

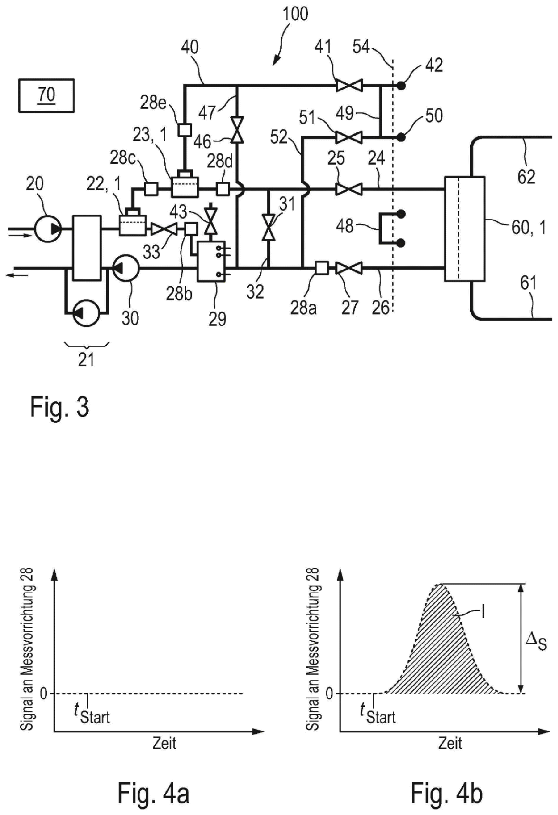

Fig. 3 zeigt eine weitere Ausführungsform einer erfindungsgemäßen extrakorporalen Blutbehandlungsvorrichtung 100.

Gegenüber der in Fig. 2 dargestellten Ausführungsform weist die in Fig. 3 dargestellte extrakorporale Blutbehandlungsvorrichtung 100 zusätzlich einen Dialysator 60 sowie eine arterielle Schlauchleitung 61 und eine venöse Schlauchleitung 62 auf. Im Übrigen besitzen die Bezugsziffern die gleiche Bedeutung wie die entsprechenden Bezugsziffern der Fig. 2.

Fig. 4a zeigt schematisch einen exemplarischen Messsignalverlauf für einen nicht erfindungsgemäßen Filter. Zum Zeitpunkt t_{Start} startet ein erstmaliges Durchspülen des Filters mit Wasser. Da ein Inneres des Filters nicht mit einer Markierungssubstanz versehen ist, ist kein Signalanstieg zu detektieren.

Fig. 4b zeigt schematisch einen exemplarischen Messsignalverlauf für einen erfindungsgemäßen Filter. **In** diesem Fall ist ein Signalanstieg an einer entsprechenden Messvorrichtung, beispielsweise an einer oder mehreren der in den Fig. 2 und 3 beschriebenen Messvorrichtungen, deutlich erkennbar. Auf diese Weise kann somit erkannt werden, ob ein erfindungsgemäßer Filter in eine extrakorporale Blutbehandlungsvorrichtung eingebaut wurde.

Anhand des Signalverlaufs kann zudem eine Aussage darüber getroffen werden, um welchen Filter es sich handelt. Ein Filter, insbesondere ein Dialysator, kann sich beispielsweise im Füllvolumen, insbesondere in Bezug auf die Blutseite und/oder Dialysatseite, in der Membranoberfläche, in der Porengröße, in dem Membranmaterial, etc. unterscheiden.

Beispielsweise ist es erfindungsgemäß denkbar, dass ein Filter mit einer größeren Menge an Markierungssubstanz versehen ist als ein anderer Filter. Dies würde sich dann im Signalverlauf dadurch bemerkbar machen, dass das Integral I oder auch die Höhe des Ausschlags Δ_{S} größer ausfallen würde.

Alternativ oder in Kombination kann ein Filter auch zwei unterschiedliche Markierungssubstanzen enthalten, die sich bezüglich ihres Absorptionsspektrums voneinander unterscheiden.

Alternativ oder in Kombination kann ein Filter zusätzlich zu lichtabsorbierenden Markierungssubstanzen ein Salz als Markierungssubstanz enthalten, dessen Menge und die daraus resultierende Konzentration bzw. Leitfähigkeit, d. h. das Integral I und/oder der Ausschlag Δ_{S}, variieren kann. Auf diese Weise können unterschiedliche Kombinationen von Markierungssubstanzen realisiert werden, wobei jede Kombination charakteristisch für einen Filter sein kann. Durch einen Vergleich mit hinterlegten Daten kann der Filter somit nicht nur erkannt, sondern auch identifiziert werden.

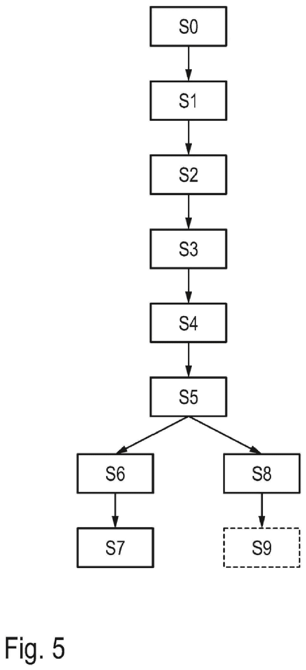

Fig. 5 zeigt schematisch ein Flussdiagramm einer Ausführungsform eines erfindungsgemäßen Verfahrens.

Bei dem Verfahren handelt es sich um ein Verfahren zum Erkennen eines erfindungsgemäßen Filters in einer extrakorporalen Blutbehandlungsvorrichtung. Das Verfahren weist die nachfolgenden Schritte auf:

| | |
|---|---|
| S1) | Einbauen, insbesondere Einwechseln, eines Filters in die extrakorporale Blutbehandlungsvorrichtung, |
| S2) | erstmaliges Durchspülen des eingebauten Filters mit einem wässrigen Fluid, |
| S3) | Aufnehmen eines Messsignals mit Hilfe einer Messeinrichtung der extrakorporalen Blutbehandlungsvorrichtung, wobei das Messsignal eine chemische und/oder physikalische Eigenschaft der Markierungssubstanz repräsentiert, |
| S4) | Auswerten des aufgenommenen Messsignals und |
| S5) | Erkennen anhand des ausgewerteten Messsignals, ob es sich bei dem eingebauten Filter um einen erfindungsgemäßen Filter handelt. |

Beim Durchführen von Schritt S1) ersetzt der eingebaute Filter vorzugsweise einen anderen Filter der extrakorporalen Blutbehandlungsvorrichtung. Mit anderen Worten wird der Schritt S1) vorzugsweise im Rahmen eines Filterwechsels vorgenommen. Alternativ kann durch den Schritt S1) erstmalig ein Filter in die extrakorporale Blutbehandlungsvorrichtung eingebaut werden.

Bei dem wässrigen Fluid handelt es sich bevorzugt um Wasser wie Reinstwasser oder Permeat, basisches Fluid, saures Fluid oder Dialysierflüssigkeit.

Bei der Messeinrichtung gemäß Schritt S3) handelt es sich vorzugsweise um eine optische Messeinrichtung, insbesondere um eine Extinktions- oder Absorptionsmesseinrichtung, oder eine Leitfähigkeitsmesseinrichtung. Vorzugsweise handelt es sich dementsprechend bei der chemischen und/oder physikalischen Eigenschaft der Markierungssubstanz um eine optische Eigenschaft, insbesondere die Extinktion oder Absorption, oder die Leitfähigkeit der Markierungssubstanz.

Das Verfahren kann ferner vor dem Schritt S1) einen weiteren Schritt S0) aufweisen:
- Ausgeben eines Aufforderungshinweises zum Filterwechsel, wenn wenigstens ein Kriterium für den Filterwechsel, d. h. wenigstens ein Wechselkriterium, erreicht ist.

Das wenigstens eine Wechselkriterium kann beispielsweise eine definierte Anzahl von mit Hilfe der extrakorporalen Blutbehandlungsvorrichtung durchzuführenden Einzelbehandlungen und/oder eine definierte Anzahl von Betriebsstunden sein.

Bevorzugt weist das Verfahren nach dem Schritt S5) ferner einen Schritt S6) auf:
- Identifizieren des Filters anhand wenigstens eines Merkmals des eingebauten Filters, wenn der Schritt S5) ergibt, dass es sich bei dem eingebauten Filter um einen erfindungsgemäßen Filter handelt.

Das wenigstens eine Merkmal des Filters kann ausgewählt sein aus der Gruppe bestehend aus Form, Größe, Füllvolumen, Porendurchmesser einer Membran des Filters, Material einer Membran des Filters, Oberfläche einer Membran des Filters und Kombinationen von wenigstens zwei der vorgenannten Merkmale.

Ferner kann das Verfahren nach dem Schritt S6) einen weiteren Schritt S7) aufweisen:
- Zurücksetzen wenigstens eines / des wenigstens einen Kriteriums für einen Filterwechsel.

Ferner kann das Verfahren nach dem Schritt S5) einen weiteren Schritt S8) aufweisen:
- Ausgeben eines Hinweises, dass der eingebaute Filter unbekannt ist und/oder gewechselt werden soll/muss, wenn der Schritt S5) ergibt, dass es sich bei dem eingebauten Filter nicht um einen erfindungsgemäßen Filter handelt.

Ferner kann das Verfahren nach dem Schritt S8) einen weiteren Schritt S9) aufweisen:
- Sperren der extrakorporalen Blutbehandlungsvorrichtung.

## Patentansprüche

1. Filter für eine extrakorporale Blutbehandlungsvorrichtung, **dadurch gekennzeichnet, dass** ein Inneres des Filters mit einer Markierungssubstanz versehen ist, die durch eine Messeinrichtung der extrakorporalen Blutbehandlungsvorrichtung nachweisbar ist.

2. Filter nach Anspruch 1, **dadurch gekennzeichnet, dass** ein Strömungsbereich des Filters, der von einem Fluid durchströmbar ist, mit der Markierungssubstanz versehen ist.

3. Filter nach Anspruch 1 oder 2, **dadurch gekennzeichnet, dass** eine Innenoberfläche, insbesondere Membranoberfläche, vorzugsweise Hohlfasermembranoberfläche, des Filters mit der Markierungssubstanz versehen ist.

4. Filter nach einem der vorhergehenden Ansprüche, **dadurch gekennzeichnet, dass** die Markierungssubstanz eine mit Hilfe eines wässrigen Fluids entfernbare, vorzugsweise eine wenigstens teilweise wasserlösliche, Substanz umfasst.

5. Filter nach einem der vorhergehenden Ansprüche, **dadurch gekennzeichnet, dass** die Markierungssubstanz ein Salz, insbesondere ein in einer Dialysierflüssigkeit und/oder im Blut vorkommendes Salz, umfasst.

6. Filter nach Anspruch 5, **dadurch gekennzeichnet, dass** das Salz ausgewählt ist aus der Gruppe bestehend aus Natriumchlorid, Kaliumchlorid, Magnesiumchlorid, Calciumchlorid, Natriumhydrogencarbonat und Mischungen von wenigsten zwei der vorgenannten Salze.

7. Filter nach einem der vorhergehenden Ansprüche, **dadurch gekennzeichnet, dass** die Markierungssubstanz einen Farbstoff umfasst.

8. Filter nach Anspruch 7, **dadurch gekennzeichnet, dass** der Farbstoff ausgewählt ist aus der Gruppe bestehend aus Chlorophyll, Harnsäure, Vitamin, Thiamin, Riboflavin, Pyridoxin, Cobalamin, Ascorbinsäure, Pantothensäure, Folsäure, Nicotinsäure, Biotin und Mischungen von wenigstens zwei der vorgenannten Farbstoffe.

9. Filter nach einem der vorhergehenden Ansprüche, **dadurch gekennzeichnet, dass** die Markierungssubstanz von einem wenigstens teilweise wasserlöslichen und insbesondere folienförmig gestalteten Material umhüllt ist.

10. Filter nach einem der vorhergehenden Ansprüche, **dadurch gekennzeichnet, dass** es sich bei dem Filter um einen Dialysierflüssigkeitsfilter handelt.

11. Filter nach einem der Ansprüche 1 bis 9, **dadurch gekennzeichnet, dass** es sich bei dem Filter um einen Dialysator handelt.

12. Extrakorporale Blutbehandlungsvorrichtung, aufweisend wenigstens einen Filter nach einem der Ansprüche 1 bis 11.

13. Verwendung eines Filters nach einem der vorhergehenden Ansprüche für eine extrakorporale Blutbehandlungsvorrichtung.

14. Verfahren zum Erkennen eines Filter nach einem der Ansprüche 1 bis 11 in einer extrakorporalen Blutbehandlungsvorrichtung, aufweisend die Schritte:
| | |
|---|---|
| S1) | Einbauen eines Filters in die extrakorporale Blutbehandlungsvorrichtung, |
| S2) | erstmaliges Durchspülen des eingebauten Filters mit einem wässrigen Fluid, |
| S3) | Aufnehmen eines Messsignals mit Hilfe einer Messeinrichtung der extrakorporalen Blutbehandlungsvorrichtung, wobei das Messsignal eine chemische und/oder physikalische Eigenschaft der Markierungssubstanz repräsentiert, |
| S4) | Auswerten des aufgenommenen Messsignals und |
| S5) | Erkennen anhand des ausgewerteten Messignals, ob es sich bei dem eingebauten Filter um einen Filter nach einem der Ansprüche 1 bis 11 handelt. |

15. Verfahren nach Anspruch 14, **dadurch gekennzeichnet, dass** das Verfahren ferner einen Schritt S6) aufweist:
- Identifizieren eines Merkmals des eingebauten Filters anhand des ausgewerteten Messsignals, wenn der Schritt S5) ergibt, dass es sich bei dem eingebauten Filter um einen Filter nach einem der Ansprüche 1 bis 11 handelt.
